# EUROPEAN PATENT APPLICATION

(11) **EP 4 566 576 A2**
(43) Date of publication of application: **11.06.2025**
(21) Application number: 24222637.1
(22) Date of filing: 11.11.2021
(51) Int. Cl.: A61F 13/02

(54) **DRESSING FOR ENHANCED RADIAL COLLAPSE**

(30) Priority: 01.12.2020 US 202063119931 P
(62) Divisional of application: 21811144.1
(71) Applicant: KCI Manufacturing Unlimited Company, Athlone, Co. Westmeath, N37XF22 (IE)
(72) Inventor: LOCKE, Christopher Brian, Bracknell, Berkshire, RG12 8HT (GB); ROBINSON, Timothy Mark, Bracknell, Berkshire, RG12 8HT (GB)
(74) Representative: Simmons & Simmons

(57) **Abstract**

A dressing for treating a tissue site with negative pressure may include a fluid management layer and a manifold layer coupled to the fluid management layer. The manifold layer may have a first surface facing the fluid management layer, a second surface opposite the first surface, and a thickness extending between the first surface and the second surface. The manifold layer may also include collapsible apertures extending at least partially through the thickness of the manifold layer from the first surface. The collapsible apertures may deform in response to a negative pressure applied to the manifold layer.

## Description

### CROSS-REFERENCE TO RELATED APPLICATIONS

This application claims the benefit of priority to U.S. Provisional Application No. 63/119,931, filed on December 1, 2020, which is incorporated herein by reference in its entirety.

### TECHNICAL FIELD

The invention set forth in the appended claims relates generally to tissue treatment systems and more particularly, but without limitation, to dressings and systems for tissue treatment with negative pressure and methods of using the dressings for tissue treatment with negative pressure.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and microdeformation of tissue at a wound site. Together, these benefits can increase development of granulation tissue and reduce healing times.

While the clinical benefits of negative-pressure therapy are widely known, improvements to therapy systems, components, and processes may benefit healthcare providers and patients.

### BRIEF SUMMARY

New and useful systems, apparatuses, and methods for treating tissue in a negative-pressure therapy environment are set forth in the appended claims. Illustrative embodiments are also provided to enable a person skilled in the art to make and use the claimed subject matter.

For example, in some embodiments, negative pressure applied through a dressing including a manifold layer may provide negative-pressure therapy to a tissue site. The negative pressure may be effective to cause the manifold layer to exhibit radial collapse such that the area of one of more surfaces of the manifold layer, when subjected to negative pressure, decrease. For example, the radial collapse may be substantially due to collapse of one or more of the collapsible apertures present within the manifold layer. In some embodiments, the radial collapse exhibited by the manifold layer 205 may have the effect of drawing the edges of the tissue site (e.g., a wound) together, for example, toward the center, which may help to reduce edema and/or to apply load to the edges of the wound, thereby improving the effectiveness of the therapy.

Also for example, in some embodiments, negative pressure applied through a dressing including a manifold layer may be formed from a closed-cell foam, such as an expanded foam. For example, the particular process by which the expanded foam is made may allow additives and modifiers, for example, an antimicrobial materials and/or a superabsorbent polymer, to be incorporated into and made integral to the polymeric material that forms the expanded foam. The incorporation of an antimicrobial materials and/or a superabsorbent polymer into an expanded foam may provide unique and beneficial properties not associated with conventional foams. Additionally, the use of an expanded foam as the manifold layer may also decrease the opportunity for any in-growth of tissue into the foam during healing of a wound or other tissue site.

In some embodiments is a dressing for treating a tissue site with negative pressure. The dressing may comprise a fluid management layer comprising a polymer film having a plurality of fluid restrictions extending through the polymer film and configured to deform. The dressing may also comprise a manifold layer coupled to the fluid management layer. The manifold layer may have a first surface facing the fluid management layer, a second surface opposite the first surface, and a thickness extending between the first surface and the second surface. The manifold layer may also comprise a plurality of collapsible apertures extending at least partially through the thickness of the manifold layer from the first surface. The collapsible apertures may be configured to deform in response to a negative pressure applied to the manifold layer. In some embodiments, the manifold layer may comprise an expanded foam material. The expanded foam material may be formed by a process comprising impregnating a polymeric material with an inert gas at high heat and pressure to form an impregnated polymeric material and expanding the impregnated polymeric material to form the expanded foam material.

Also, in some embodiments is a dressing for treating a tissue site with negative pressure. The dressing may comprise a fluid management layer comprising a polymer film having a plurality of fluid restrictions extending through the polymer film and configured to deform. The dressing may also comprise a manifold layer coupled to the fluid management layer. The manifold layer may have a first surface facing the fluid management layer, a second surface opposite the first surface, and a thickness extending between the first surface and the second surface. The manifold may comprise an expanded foam material. The expanded foam material may be formed by a process comprising impregnating a polymeric material with an inert gas at high heat and pressure to form an impregnated polymeric material and expanding the impregnated polymeric material to form the expanded foam material.

### BRIEF DESCRIPTION OF THE DRAWINGS

Figure 1 is a block diagram of an example embodiment of a therapy system that can provide tissue treatment in accordance with this specification;
Figure 2 is an exploded view of an example of a dressing, illustrating additional details that may be associated with some example embodiments of the therapy system of Figure 1;
Figure 3 is a schematic view of an example configuration of a layer that may be associated with some embodiments of the dressing of Figure 2;
Figure 4 is a detailed view of the layer of Figure 3;
Figure 5 is a detailed view of the layer of Figure 3, exhibiting a response to the application of a negative pressure;
Figure 6 is a perspective view of the layer of Figure 3;
Figure 7 is a schematic view of an example configuration of fluid restrictions in a layer that may be associated with some embodiments of the dressing of Figure 2;
Figure 8 is a schematic view of an example configuration of apertures in another layer, illustrating additional details that may be associated with some embodiments of the dressing of Figure 2;
Figure 9 is a schematic view of the example layer of Figure 7 overlaid on the example layer of Figure 8;
Figure 10 is a schematic view of another example of another dressing layer, illustrating additional details that may be associated with some embodiments; and
Figure 11 and Figure 12 illustrate other example configurations of fluid restrictions that may be associated with some embodiments of layers of the dressing of Figure 2.

### DESCRIPTION OF EXAMPLE EMBODIMENTS

The following description of example embodiments provides information that enables a person skilled in the art to make and use the subject matter set forth in the appended claims, and may omit certain details already well-known in the art. The following detailed description is, therefore, to be taken as illustrative and not limiting.

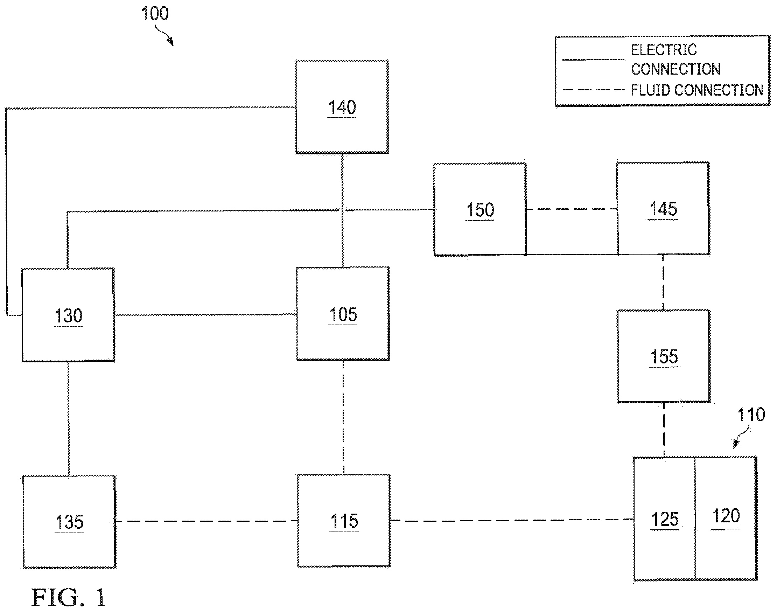

Figure 1 is a block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partialthickness bums, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of Figure 1, the dressing 110 may comprise a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

The therapy system 100 may also include a source of instillation solution. For example, a solution source 145 may be fluidly coupled to the dressing 110, as illustrated in the example embodiment of Figure 1. The solution source 145 may be fluidly coupled to a positive-pressure source such as a positive-pressure source 150, a negative-pressure source such as the negative-pressure source 105, or both in some embodiments. A regulator, such as an instillation regulator 155, may also be fluidly coupled to the solution source 145 and the dressing 110 to ensure proper dosage of instillation solution (e.g. saline) to a tissue site. For example, the instillation regulator 155 may comprise a piston that can be pneumatically actuated by the negative-pressure source 105 to draw instillation solution from the solution source 145 during a negative-pressure interval and to instill the solution to the dressing 110 during a venting interval. Additionally or alternatively, the controller 130 may be coupled to the negative-pressure source 105, the positive-pressure source 150, or both, to control dosage of instillation solution to a tissue site. In some embodiments, the instillation regulator 155 may also be fluidly coupled to the negative-pressure source 105 through the dressing 110, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130, the solution source 145, and other components into a therapy unit.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micropump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or be formed from, for example, an elastomeric film or membrane that can provide a seal adequate to maintain a negative pressure at a tissue site for a given negative-pressure source. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis, Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inspire 2327 polyurethane films, commercially available from Exopack Advanced Coatings, Wrexham, United Kingdom. In some embodiments, the cover 125 may comprise an Inspire 2301 having an MVTR (upright cup technique) of 2600 g/m²/24 hours and a thickness of about 30 microns.

An attachment device may be used to attach the cover 125 to an attachment surface, such as undamaged epidermis, a gasket, or another cover. The attachment device may take many forms. For example, an attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 145 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

In general, exudate and other fluid flow toward lower pressure along a fluid path. Thus, the term "downstream" may refer to a location in a fluid path relatively closer to a source of negative pressure or further away from a source of positive pressure. Conversely, the term "upstream" may refer to a location relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source.

Negative pressure applied across the tissue site through the tissue interface 120 in the sealed therapeutic environment can induce macro-strain and micro-strain in the tissue site. Negative pressure can also remove exudate and other fluid from a tissue site, which can be collected in the container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, the controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

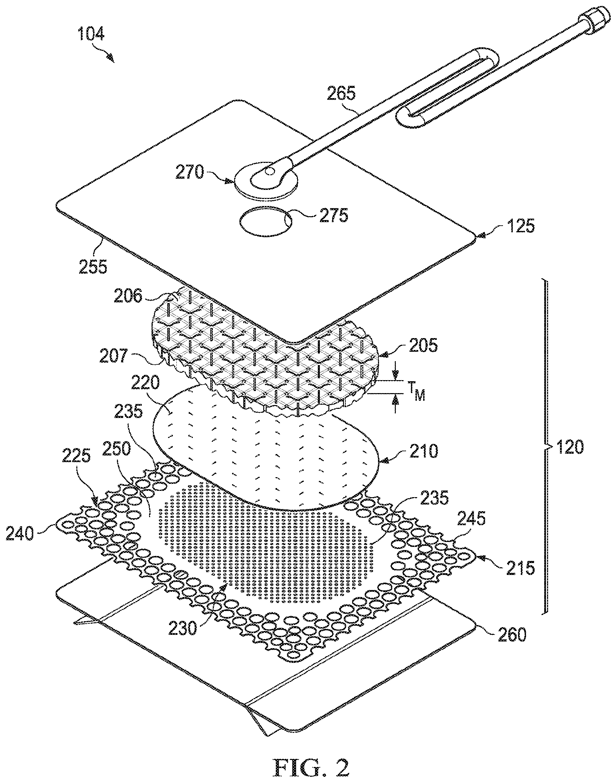

Figure 2 is an exploded view of an example of the dressing 110 of Figure 1, illustrating additional details that may be associated with some embodiments in which the tissue interface 120 comprises more than one layer. In the embodiment of Figure 2, the tissue interface 120 includes a plurality of layers, for example, a first layer, a second layer, and a third layer. More particularly, in the example of Figure 2, the tissue interface 120 comprises a manifold layer 205, a fluid management layer 210, and a contact layer 215. In some embodiments, the manifold layer 205 may be disposed adjacent to the fluid management layer 210, and the contact layer 215 may be disposed adjacent to the fluid management layer 210 opposite the manifold layer 205. For example, the manifold layer 205, the fluid management layer 210, and the contact layer 215 may be stacked so that the manifold layer 205 is in contact with the fluid management layer 210, and the fluid management layer 210 is in contact with the manifold layer 205 and the contact layer 215. One or more of the manifold layer 205, the fluid management layer 210, and the contact layer 215 may also be bonded to an adjacent layer in some embodiments.

The manifold layer 205 may comprise or provide a means for collecting or distributing fluid across the tissue interface 120 under negative pressure. For example, the manifold layer 205 may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid, such as from a source of instillation solution, across the tissue interface 120. In some illustrative embodiments, the manifold layer 205 may comprise a plurality of pathways, for example, which can be interconnected to improve distribution or collection of fluids. For example, in some embodiments, the manifold layer 205 may comprise or be formed from a porous material having interconnected fluid pathways.

In the embodiment of Figure 2, the manifold layer 205 comprises a first surface 206, a second surface 207, and a thickness *T_{M}* extending between the first surface 206 and the second surface 207. In various embodiments, the thickness of the manifold layer 205 may also vary according to needs of a prescribed therapy. The thickness *T_{M}* of the manifold layer 205 can vary according to the needs of a particular therapy. For example, in some embodiments the manifold layer 205 may have a thickness *T_{M}* in a range of about 2 millimeters to 15 millimeters, or in a range of about 3 millimeters to about 10 millimeters, or in a range of about 6 to about 8 millimeters. In the embodiment of Figure 2, the second surface 207 may face the fluid management layer 210 and the first surface 206 may be opposite the second surface 207.

In some embodiments, the manifold layer 205 also comprises a plurality of apertures extending into the thickness *T_{M}* from, the first surface 206, the second surface 207, or between the first surface 206 and the second surface 207. One or more of the apertures may be characterized with respect to one or more of its length, its width, and/or its depth. The length of an aperture may refer to a major dimension in a plane generally parallel to the first surface 206 and/or the second surface 207. The width may refer to a dimension in the plane generally parallel to the first surface 206 and/or the second surface 207 and perpendicular to the length. The depth may refer to a dimension perpendicular to the plane generally parallel to the first surface 206, as measured from the first surface 206, the second surface 207, or both.

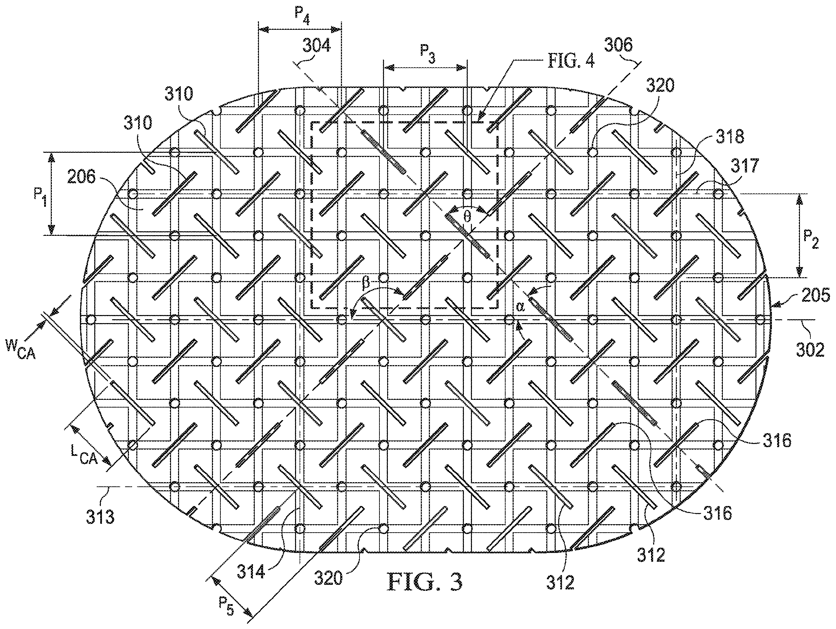

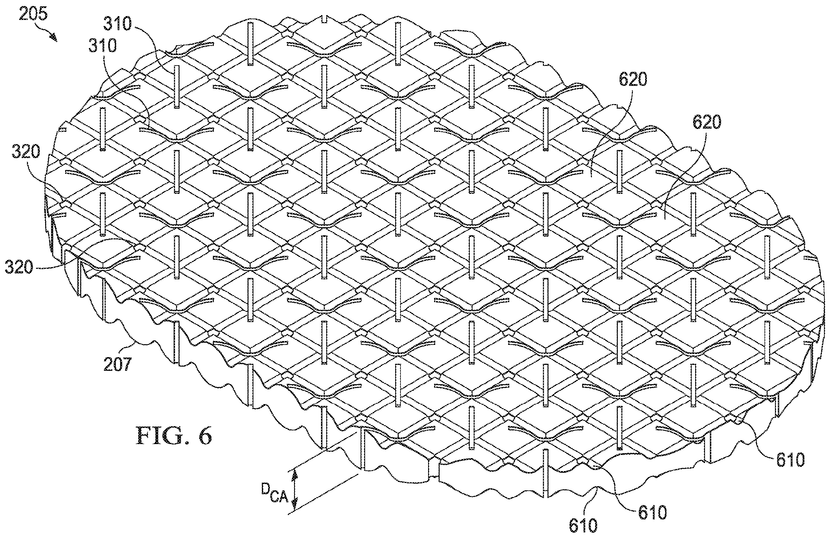

For example, referring to Figure 3, in some embodiments the manifold layer 205 comprises a plurality of collapsible apertures 310 extending into the thickness *T_{M}* from either the first surface 206 or the second surface 207. The collapsible apertures 310 may be characterized with respect to a length *L_{CA}*, a width *W_{CA}*, and a depth *D_{CA}* (shown in Figure 6). The collapsible aperture 310 may be an aperture generally configured to exhibit a relatively high degree of deformation in response to a negative pressure applied to the manifold layer 205. For example, the collapsible apertures 310 may be configured to exhibit a relatively high percentage change in the area of a cross-section in a plane parallel to either the first surface 206 or the second surface 207. For example, in response to negative pressure applied to the manifold layer 205, the collapsible apertures 310 may be configured to deform so as to exhibit a decrease in the cross-section in the plane parallel to either the first surface 206 or the second surface 207, for example, as a function of a decrease to the length *L_{CA}* and/or the width *W_{CA}* thereof. For example, the cross-sectional area of one of the collapsible apertures 310 may decrease to about 90% of the cross-sectional area of that collapsible aperture 310 in the absence of the negative pressure, or about 80%, or about 70%, or about 60%, or about 50%, or about 40%, or about 35%, or about 30%, or about 25%, or about 20%, or about 15%, or about 10% of the cross-sectional area of the collapsible aperture 310 in the absence of the negative pressure.

In various embodiments, the collapsible apertures 310 may comprise any suitable shape configured to deform upon application of the negative pressure to the manifold layer 205. For example, in various embodiments, one or more of the collapsible apertures 310 may be characterized as having a shape in a plane perpendicular to the depth *D_{CA}* of the collapsible aperture 310 that is rectangular, elongate, diamond-shaped, square, oval, ovoid, irregular, polygonal (for example, hexagonal), or amorphous.

In some embodiments, one or more of the plurality of the collapsible apertures 310 may have a length *L_{CA}* from about 4 mm to about 20 mm, or from about 8 mm to about 16 mm, or from about 10 mm to about 15 mm, or from about 12 mm to about 15 mm, or from about 10 mm to about 14 mm, or from about 12 mm to about 14 mm. Additionally or alternatively, in some embodiments, one or more of the plurality of the collapsible apertures 310 may have a width *W_{CA}* from about 1 mm to about 6 mm, or from about 1.5 mm to about 5 mm, or from about 2 mm to about 4 mm, or from about 2.5 mm to about 3.5 mm, or about 3 mm. For example, one or more of the plurality of the collapsible apertures 310 may have a length *L_{CA}* that is at least about 120% of the width *W_{CA}*, or at least about 140% of the width *W_{CA}*, or at least about 160% of the width *W_{CA}*, or at least about 180% of the width *W_{CA}*, or at least about 200% of the width *W_{CA}*, or at least about 225% of the width *W_{CA}*, or at least about 250% of the width *W_{CA}*, or at least about 275% of the width *W_{CA}*, or at least about 300% of the width *W_{CA}*, or at least about 325% of the width *W_{CA}*, or at least about 350% of the width *W_{CA}*, or at least about 375% of the width *W_{CA}*, or at least about 400% of the width *W_{CA}*, or at least about 425% of the width *W_{CA}*, or at least about 450% of the width *W_{CA}*, or at least about 475% of the width *W_{CA}*, or at least about 500% of the width *W_{CA}*, or at least about 550% of the width *W_{CA}*, or at least about 600% of the width *W_{CA}*, or at least about 650% of the width *W_{CA}*, or at least about 700% of the width *W_{CA}*, or at least about 750% of the width *W_{CA}*, or at least about 800% of the width *W_{CA}*, or at least about 850% of the width *W_{CA}*, or at least about 900% of the width *W_{CA}*, or at least about 950% of the width *W_{CA},* or at least about 1000% of the width *W_{CA}*, or at least about 1250% of the width *W_{CA}*, or at least about 1500% of the width *W_{CA}.*

One or more of the collapsible apertures 310 may be characterized with respect to the orientation of the collapsible aperture 310, for example, with respect to a line parallel to the length *L_{CA}* of the collapsible aperture 310. In some embodiments, the manifold layer 205 may include collapsible apertures 310 having a first orientation and collapsible apertures 310 having a second orientation, which may be different from the first orientation. For example, as shown in the embodiment of Figure 3, the manifold layer 205 may comprise a first portion 312 of the collapsible apertures 310 and a second portion 316 of the collapsible apertures 310. The first portion 312 of the collapsible apertures 310 may be disposed in the manifold layer 205 in a first angle or orientation α, as shown by reference to a first orientation line 304 parallel to the length *L_{CA}* of the first portion 312 of the collapsible apertures 310 and a reference line 302 generally parallel to the length of the manifold layer 205. Also, the second portion 316 of the plurality of the collapsible apertures 310 may be disposed in the manifold layer 205 in a second angle or orientation *β*, as shown by reference to a second orientation line 306 parallel to the length *L_{CA}* of the second portion 316 of the collapsible apertures 310 and the reference line 302 generally parallel to the length of the manifold layer 205. In various embodiments, the first orientation α, may range from about 0 degrees to about 180 degrees and the second orientation *β* may range from about 45 degrees to about 315 degrees.

In some embodiments, the first orientation *α* and the second orientation *β* may be such that an angle of deviation *θ* between the first orientation α and the second orientation *β* is from about 50 degrees to about 130 degrees, or from about 60 degrees to 120 degrees, or from about 70 degrees to about 110 degrees, or from about 75 degrees to about 105 degrees, or from about 80 degrees to about 100 degrees, or from about 85 degrees to about 95 degrees. In some embodiments, the angle of deviation *θ* may be about 90 degrees, for example, such that the first orientation *α* may be perpendicular or substantially perpendicular to the second orientation *β*.

Additionally or alternatively, in various embodiments, the first portion 312 of the collapsible apertures 310 may be disposed in the manifold layer 205 in one or more first rows 313 and/or first columns 314. Also, the second portion 316 of the collapsible apertures 310 may be disposed in the manifold layer 205 in one or more second rows 317 and/or second columns 318.

In some embodiments, two or more of the collapsible apertures 310 may also be characterized by a pitch, which may refer to a spacing between corresponding points of the two respective collapsible apertures as disposed within the manifold layer 205. For example, in some embodiments the pitch may indicate the spacing between the respective centroids of two or more collapsible apertures 310. Some patterns of the collapsible apertures 310 may be characterized by a single pitch value, while other patterns of the collapsible apertures 310 may be characterized by at least two pitch values. For example, if the spacing between centroids of the collapsible apertures 310 is the same in all orientations, the pitch may be characterized by a single value indicating the spacing between centroids in adjacent rows. In some embodiments, the pattern comprising the first portion 312 of the collapsible apertures 310 and the second portion 316 of the collapsible apertures 310 may be characterized by five pitch values, *P₁, P₂*, *P₃*, *P₄*, and *P₅. P₁* may be the spacing between the centroids of two of the first portion 312 of the collapsible apertures 310 in adjacent first rows 313 in a direction perpendicular to the first rows 313. *P₂* may be the spacing between the centroids of each of the second portion 316 of the collapsible apertures 310 in adjacent second rows 317 in a direction perpendicular to the second rows 317. *P₃* may be the spacing between adjacent centroids of two collapsible apertures 310 of the first portion 312 of the collapsible apertures 310 within each the first rows 313 and parallel to the first rows 313. *P₄* may be the spacing between adjacent centroids of two of the collapsible apertures 310 of the second portion 316 of the collapsible apertures 310 within each of the second rows 317 and parallel to the second rows 317. *P₅* may be the spacing between the centroid of one of the first portion 312 of the collapsible apertures 310 and the centroid of one of the second portion 316 of the collapsible apertures 310 in adjacent rows. In some embodiments, *P₁* and *P₂* may be substantially equal and *P₃* and *P₄* may be substantially equal, within acceptable manufacturing tolerances. In some embodiments, for example, where *P₁* is substantially equal to *P₂ (e.g., P₁* = *P₂*)*,* then *P₅* may be equal to about half of *P₁* each of and *P₂* (e.g., *P₅*=0*.*5 x *P₁*=0.5 x *P₂*). For example, in some embodiments, *P₁* and *P₂* may be about 16 millimeters to about 18 millimeters, *P₃* and *P₄* may be about 8 millimeters, and P5 may be about 7 millimeters to about 10 millimeters.

Also, in some embodiments, the first portion 312 of the collapsible apertures 310 may be characterized with respect to their alignment to one or more of the second portion 316 of the collapsible apertures 310; likewise, the second portion 316 of the collapsible apertures 310 may be characterized with respect to their alignment to one or more of the first portion 312 of the collapsible apertures 310. In some embodiments, the alignment of the first portion 312 of the collapsible apertures 310 with respect to the second portion 316 of the collapsible apertures 310 and/or the alignment of the second portion 316 of the collapsible apertures 310 with respect to the first portion 312 of the collapsible apertures 310 may be configured to cause the one or more of the plurality of the collapsible apertures 310 to be deformed upon the application of negative pressure to the manifold layer 205.

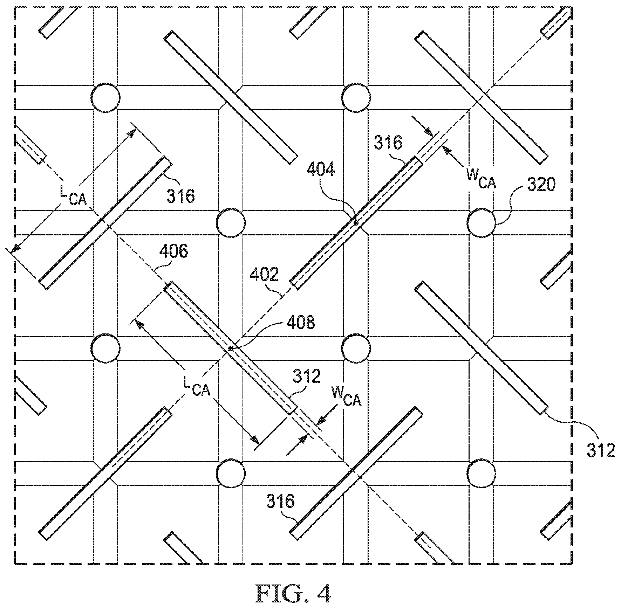

For example, referring to Figure 4, a detailed view of a portion of the manifold layer 205 is illustrated. In the embodiment of Figure 4, a first perpendicular bisector 402 of the length *L_{CA}* (e.g., a line extending through the midpoint of the length *L_{CA}*, perpendicular to the length *L_{CA}*) of one of the first portion 312 of the collapsible apertures 310 may be bounded by or intersect a portion of the width *W_{CA}* of one or more of the second portion 316 of the collapsible apertures 310. For example, the first perpendicular bisector 402 may pass through a midpoint 404 of the width *W_{CA}* of one or more of the second portion 316 of the collapsible apertures 310.

Additionally or alternatively, and as also shown in the embodiment of Figure 4, a second perpendicular bisector 406 of the length *L_{CA}* of one of the second portion 316 of the collapsible apertures 310 may be bounded by or intersection of portion of the width *W_{CA}* of one or more of the first portion 312 of the collapsible apertures 310. For example, the second perpendicular bisector 406 may pass through a midpoint 408 of the width *W_{CA}* of one or more of the first portion 312 of the collapsible apertures 310.

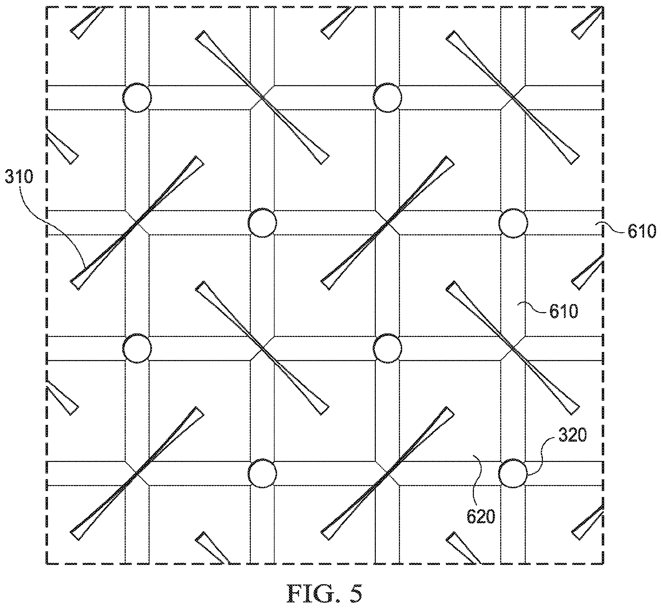

One or more of the plurality of the collapsible apertures 310 may be characterized with respect to its response to a negative pressure. For example, in response to the application of a negative pressure to the manifold layer 205, one or more of the collapsible apertures 310 may be characterized as exhibiting a decrease in the area of the cross-sectional plane perpendicular to the depth *D_{CA}* (shown in Figure 6) of the collapsible aperture 310, for example, as a function of a decrease to the length *L_{CA}* and/or the width *W_{CA}* thereof. More particularly, in some embodiments, the decrease in the area of the cross-sectional plane perpendicular to the depth *D_{CA}* of the collapsible aperture 310 may be predominantly due to a decrease in the width *W_{CA}* of the collapsible aperture 310. For example, referring to Figure 5, an example of a portion of the manifold layer 205 is shown exhibiting a response to negative pressure. In the example of Figure 5, the collapsible apertures 310 exhibit a decrease in the area of the cross-sectional plane perpendicular to the depth *D_{CA}* of the collapsible aperture 310, in particular, substantially due to a decrease in the width *W_{CA}* of the collapsible apertures 310.

Additionally, in some embodiments the manifold layer 205 may be characterized as exhibiting radial collapse. Radial collapse may refer to a deformation of the manifold layer 205 or a portion thereof such that the manifold layer 205 exhibits a decrease in at least one dimension of the first surface 206 and/or the second surface 207 relative to a nominal or relaxed length and/or width, when the manifold layer 205 is not subjected to negative pressure. The radial collapse exhibited by the manifold layer 205 may be determined by various factors related to the collapsible apertures 310, for example, the dimension of the collapsible apertures (e.g., the length *L_{CA},* the width *W_{CA}*, and the size relationship between the length *L_{CA}* and width *W_{CA}*), the relationship between two of the collapsible apertures 310 (e.g., the pitch, orientation, and/or alignment between two or more collapsible apertures 310), and combinations thereof. In various embodiments, manipulation of one or more of these parameters may be effective to vary the radial collapse exhibited by the manifold layer 205 to meet the needs of a particular therapy. For example, in various embodiments the manifold layer 205 may be characterized as exhibiting radial collapse such that the area of the first surface 206 and/or the second surface 207 when subjected to negative pressure is not more than about 90% of the area of the respective surface when the manifold layer 205 is not subjected to negative pressure, or not more than about 80%, or not more than about 70%, or not more than about 60%, or not more than about 50% of the area of the respective surface when the manifold layer 205 is not subjected to negative pressure.

For example, in some embodiments where the width *W_{CA}* of the collapsible apertures 310 may substantially define a proportion of collapse exhibited by the plurality of the collapsible apertures 310, the width *W_{CA}* of the collapsible apertures 310 may also, at least in part, determine the overall radial collapse demonstrated by the manifold layer 205.

Also, in some embodiments where the width *W_{CA}* of the collapsible apertures 310 may substantially define a proportion of collapse exhibited by the plurality of the collapsible apertures 310, the compressibility of the manifold layer 205 may increase in a direction as the orientation (e.g., the length) of the collapsible aperture 310 approaches perpendicular to that direction. For example, as the first orientation *α* of the first portion 312 of the collapsible apertures 310, shown by reference to the first orientation line 304, approaches the orientation of the reference line 302 generally parallel to the length of the manifold layer 205 and/or as the second orientation *β* of the second portion 316 of the collapsible apertures 310, as shown by reference to a second orientation line 306, approaches the orientation of the reference line 302 generally parallel to the length of the manifold layer 205, the compressibility of the manifold layer 205 perpendicular to the reference line 302 may increase. Consequently, if negative pressure is applied to the manifold layer 205, the manifold layer 205 may contract more in one direction than another.

Additionally or alternatively, referring again to the embodiment of Figure 3, in some embodiments the manifold layer 205 may comprise a plurality of fluid apertures 320 extending through the thickness *T_{M}* between the first surface 206 and the second surface 207. One or more of the fluid apertures 320 may include or be an aperture generally configured to exhibit a relatively low degree of deformation in response to a negative pressure applied to the manifold layer 205, for example, to exhibit a relatively low percentage change in a cross-section in a plane parallel to either the first surface 206 or the second surface 207. For example, in response to negative pressure applied to the manifold layer 205, the fluid apertures 320 may be configured to exhibit limited deformation such that the cross-section in the plane parallel to either the first surface 206 or the second surface 207 retains at least to about 90% of the cross-sectional area in the absence of the negative pressure, or at least about 80%, or at least about 70%, or at least about 60%, or at least about 50% of the cross-sectional area in the absence of the negative pressure. Generally, the fluid apertures 320 may be configured to remain open to allow for the communication of a fluid between the first surface 206 and the second surface 207.

In various embodiments, the fluid apertures 320 may comprise any suitable shape or combination of shapes configured to withstand deformation upon application of the negative pressure to the manifold layer 205. For example, in various embodiments, one of more of the fluid apertures 320 may be characterized as having a shape that is circular, rectangular, diamond-shaped, square, oval, ovoid, irregular, polygonal (for example, hexagonal), or amorphous. In some embodiments, in comparison to the collapsible apertures 310, the fluid apertures 320 may be characterized as having a cross-sectional shape that does not deviate by more than about 10%, or about 20%, or about 30%, or about 40%, or about 50%, or about 60%, about 70%, or about 80%, or about 90%, or about 100% when subjected to negative pressure.

In some embodiments, the fluid apertures 320 may be disposed in the manifold layer 205 in a suitable pattern, for example, in a plurality of rows and/or columns. For example, in the embodiment of Figure 3, the fluid apertures 320 may be interposed between the collapsible apertures 310 of the first portion 312 of the collapsible apertures 310, for example, within the first rows 313 and the first columns 314. Also, in the embodiment of Figure 3, the fluid apertures 320 may be interposed between the collapsible apertures 310 of the second portion 316 of the collapsible apertures 310, for example, within the second rows 317 and the second columns 318. In some embodiments, one of the fluid apertures 320 may be disposed substantially equidistant between two collapsible apertures 310 of a given row or column.

The plurality of the collapsible apertures 310 and the plurality of the fluid apertures 320 may collectively form a percent open area the manifold layer 205. The percent open area of the manifold layer 205 may be equal to the percentage of sum of the area of the plurality of the plurality of the collapsible apertures 310 and fluid apertures 320 relative to the area of the manifold layer 205. In some embodiments, the percent open area may be between about 40% and about 60%. In other embodiments, the percent open area may be about 56%.

In some embodiments, the first surface 206 and/or the second surface 207 may be generally characterized as planar surfaces, for example, although not necessarily perfectly flat, being generally recognizable as flat or capable of being laid flat. For example, a planar surface may include minor undulations and/or deviations from a single geometric plane.

Additionally or alternatively, in some embodiments the first surface 206 and/or the second surface 207 may comprise a sculpted surface; for example, comprising one or more surface features. For example, in some embodiments the first surface 206 and the second surface 207 may comprise one or more features characterized as grooves, furrows, hollows, passageways, or channels. For example, referring to the embodiment of Figure 6, the manifold layer 205 may comprise a plurality of channels 610 disposed within the first surface 206. The channels 610 may have any suitable size and configuration. For example, in various embodiments the channels 610 may be characterized as having straight side-walls and a square or rectangular cross-section or curved side-walls. In various embodiments, the channels 610 may have any suitable spacing and may intersect at a suitable angle. For example, in the embodiment of Figure 6 the channels 610 may generally form a grid pattern. For example, a first portion of the channels 610 may deviate from a second portion of the channels 610 such that the first and second portion of the channels 610 may intersect, for example, at an angle between about 45 degrees and about 135 degrees, or for example, at about 90 degrees.

In some embodiments, the plurality of the channels 610 may cooperatively define one or more additional surface features, for example, a plurality of pillars 620 substantially bounded by the channels 610.

In some embodiments, one or more of the channels 610 may be disposed within the first surface 206 and/or the second surface 207 such that the channels 610 may provide a route of fluid communication to or from the collapsible apertures 310 and/or the fluid apertures 320. For example, one or more of the channels 610 may be aligned with one or more of the collapsible apertures 310, one or more of the fluid apertures 320, or one or more of both the collapsible apertures 310 and the fluid apertures 320. In the embodiment of Figure 6, one of the first portion of the channels 610 may intersect one of the second portion of channels 610 at a location aligned with each of the collapsible apertures 310 and the fluid apertures 320. In such embodiments, the channels 610 may further contribute to the distribution of fluids via the manifold layer 205, for example, both through the manifold layer 205 and across the first surface 206 and/or the second surface 207 of the manifold layer 205.

In some embodiments, one or more of the collapsible apertures 310, the fluid apertures 320, or various surface features of the manifold layer 205 such as the channels 610 or pillars 620, may be disposed in the manifold layer 205 by any suitable process, an example of which includes thermoforming. Thermoforming of a foam may involve embossing patterns and structures, such as apertures or channels, into the surfaces of the foam under heat and/or pressure.

In some embodiments, the manifold layer 205 may comprise or be formed from a reticulated foam having pore sizes and free volume that may vary according to needs of a prescribed therapy. In some embodiments, the reticulated foam may comprise one or more of a polyol, such as polyester or polyether, an isocyanate, such as toluene and diisocyanate, and polymerization modifiers such as amines and tin compounds. In one non-limiting example, the manifold layer 205 may be a reticulated polyurethane ether foam such as used in a V.A.C.^{®} GRANUFOAM^{™} Dressing or a V.A.C.^{®} VERAFLO^{™} Dressing, both available from Kinetic Concepts, Inc. of San Antonio, Texas.

In some embodiments, the manifold layer 205 may comprise a felted foam, for example, a foam formed by a felting process. Any porous foam suitable for felting may be used, including the example foams mentioned herein, such as GRANUFOAM^{™} Dressing. In some embodiments, the felting process comprises a thermoforming process that permanently compresses a foam to increase the density of that foam while maintaining interconnected pathways. Felting may be performed by any known methods, which may include applying heat and pressure to a porous material or foam material. Some felting methods may include compressing a foam blank, for example, a reticulated foam blank, between one or more heated platens or dies for a specified period of time and at a specified temperature. The direction of compression may be along the thickness of the foam blank.

The period of time of compression may range from 10 minutes up to 24 hours, though the time period may be more or less depending on the specific type of porous material used. Further, in some examples, the temperature may range between 120°C to 260°C. Generally, the lower the temperature of the platen, the longer a porous material must be held in compression. After the specified time period has elapsed, the pressure and heat will form a felted structure or surface on or through the porous material or a portion of the porous material. In some embodiments, multiple iterations of compressing and heating may be performed. For example, the felting process may comprise two, three, four, five, six, seven, eight, or more iterations of heating and compression.

Not intending to be bound by theory, the felting process may alter certain properties of the original material, including pore shape and/or size, elasticity, density, and density distribution. For example, struts that define pores in the foam may be deformed during the felting process, resulting in flattened pore shapes. The deformed struts can also decrease the elasticity of the foam. The density of the foam is generally increased by felting. In some embodiments, contact with hot-press platens in the felting process can also result in a density gradient in which the density is greater at the surface and the pores size is smaller at the surface. In some embodiments, the felted structure may be comparatively smoother than an unfinished or non-felted surface or portion of the porous material. Further, the pores in the felted structure may be smaller than the pores throughout any unfinished or non-felted surface or portion of the porous material. In some examples, the felted structure may be imparted to all surfaces or portions of the porous material. Further, in some examples, the felted structure may extend into or through an entire thickness of the porous material such that the all of the porous material is felted.

A felted foam may be characterized with respect to a firmness factor, which is indicative of the compression of the foam. The firmness factor of a felted foam can be specified as the ratio of original thickness to final thickness. In some embodiments, the felted foam of the manifold layer 205 may have a firmness factor greater than 1. The degree of compression may affect the physical properties of the felted foam. For example, felted foam has an increased effective density compared to a foam of the same material that is not felted. The felting process can also affect fluid-to-foam interactions. For example, as the density increases, compressibility or collapse may decrease. Therefore, foams which have different compressibility or collapse may have different firmness factors. In some example embodiments, a firmness factor can range from about 2 to about 10, preferably about 3 to about 5. For example, the firmness factor of the manifold layer 205 felted foam may be about 5 in some embodiments. There is a general, linear relationship between firmness level, density, pore size (or pores per inch) and compressibility. For example, a foam that is felted to a firmness factor of 3 will show a three-fold density increase and compress to about a third of its original thickness.

In some embodiments, one or more suitable foam blanks may be used for forming the manifold layer 205. The foam blank(s) may have about 40 to about 50 pores per inch on average, a density of about 1.3 to about 1.6 lb/ft³, a free volume of about 90% or more, an average pore size in a range of about 400 to about 600 microns, a 25% compression load deflection of at least 0.35 pounds per square inch, and/or a 65% compression load deflection of at least 0.43 pounds per square inch. In some embodiments, the foam blank(s) may have a thickness greater than 10 millimeters, for example 10-35 millimeters, 10-25 millimeters, 10-20 millimeters, or 15-20 millimeters. In some embodiments, the foam blank(s) may be felted to provide denser foam for the manifold layer 205. For example, one or more foam blanks may be felted to a firmness factor of 2-10 to form the manifold layer 205. In some embodiments, the foam blank may be felted to a firmness factor of from about 3 to about 7, for example, a firmness factor of about 5.

Alternatively, in some embodiments, the manifold layer 205 may comprise a closed-cell foam. For example, in some embodiments, the manifold layer 205 may comprise an expanded foam, for example, a foam formed from a process by which comprises expansion of a foam precursor material. For example, in some embodiments an expanded foam may be formed from a process comprising extrusion of a polymeric material, impregnation of the polymeric material with an inert gas at high heat and pressure to form an impregnated polymeric material, and expansion of the impregnated polymeric material to form the expanded foam material.

During the extrusion step, raw polymeric material is melted and forced through a die to form a generally continuous stock material, for example, the extruded polymeric material. The polymeric material may comprise any suitable polymer, copolymer, or combination thereof, dependent upon the needs of a prescribed therapy. For example, in various embodiments, the polymeric material comprises cross-linked ethylene-vinyl acetate copolymer, a cross-linked polyolefin, for example, cross-linked polyethylene, or a cross-linked ethyl-methyl-acrylate copolymer.

In some embodiments, the polymeric material may have one or more additives or modifiers incorporated in an amount effective to impart a desired effect during the extrusion step. For example, in some embodiments, an antimicrobial material may be incorporated within the polymeric material during extrusion. Suitable examples of an antimicrobial material include a metal, such as silver, which may be present in metallic form, in ionic form (e.g., a silver salt), or both. In some embodiments, silver may be present in combination with one or more additional metals, for example, gold, platinum, ferro-manganese, copper, zinc, or combinations thereof. In some examples, silver may be incorporated into the polymeric material in an amount from about 1% to about 10% by weight of the polymeric material.

Additionally or alternatively, in some embodiments a superabsorbent polymer (SAP) may be incorporated within the polymeric material during extrusion. Generally, relative to their mass, SAPs can absorb and retain large quantities of liquid, and in particular water. Many medical disposables, such as canisters and dressings, use SAPs to hold and stabilize or solidify wound fluids. The SAPs may be of the type often referred to as "hydrogels," "super-absorbents," or "hydrocolloids." For example, SAPs may absorb liquids by bonding with water molecules through hydrogen bonding. In some examples, a SAP may be incorporated into the polymeric material in an amount from about 10% to about 20% by weight of the polymeric material.

During the impregnation step, the polymeric material is exposed to an inert gas under elevated heat and pressure, causing the inert gas to permeate the polymeric material. The inert gas may comprise nitrogen gas, for example, at least 90% nitrogen gas, or at least 95% nitrogen gas, or at least 99% nitrogen gas, by weight. The parameters associated with the impregnation step, for example, the temperature, partial pressure of the inert gas and the duration of the impregnation, may be manipulated to alter the properties of the impregnated polymeric material and, accordingly, the properties of the resultant expanded foam.

During the expansion step, the impregnated polymeric material is subjected to heat in the presence of a reduced pressure, for example, a pressure that is less than the pressure employed during the impregnation step. In some embodiments, the impregnated polymeric material may be expanded in a low-pressure autoclave. Not intending to be bound by theory, during the expansion step, the reduction in pressure may allow the inert gas to expand, causing the formation of pores or cells within the expanded polymeric material. The parameters associated with the expansion step, for example, the temperature, pressure, and the duration of the expansion, may be manipulated to alter the properties of the expanded polymeric material, the expanded foam.

In some embodiments, a closed-cell foam such as an expanded foam may comprise a plurality of pores or cells that can be generally characterized as not being interconnected. In some embodiments, an expanded foam may be characterized as resilient such that in the presence of a negative pressure, the expanded foam exhibits a resistance to compression, for example, a resistance to compression that is relatively high in comparison to an open-cell foam. Not intending to be bound by theory, were the expanded foam too soft, it might collapse in the presence of negative pressure such that the expanded foam would not manifold the negative pressure.

The resistance to compression exhibited by the expanded foam may be dependent upon, among other parameters, the density of the expanded foam and the hardness of the material forming the expanded foam, as well as the closed-cell nature of the expanded foam. For example, the expanded foam may be characterized as having a density of from about 0.04 g/cm^3 to about 0.06 g/cm^3 according to ISO 7214:2012, or from about 0.045 g/cm^3 to about 0.055 g/cm^3, about 0.05 g/cm^3. Additionally, the expanded foam may be characterized as having a Shore Hardness on the OO Scale of from about 40 to 55 according to ISO 868:2003, or from about 42 to about 48, or about 46. In some embodiments, the expanded foam may be characterized as exhibiting a compression stress-strain at 25% compression of about 39 for a 25 mm cell-cell according to ISO 7214:2012 and/or a compression stress-strain at 50% compression of about 100 for a 25 mm cell-cell according to ISO 7214:2012.

In some embodiments, the manifold layer 205 may comprise a closed-cell cross-linked polyolefin foam such as one of the AZOTE^{®} range of foams available from Zotefoams Plc, of London, England. In various non-limiting examples, the manifold layer 205 may comprise a closed-cell cross-linked polyethylene foam such as one of the Plastazote^{®} line of foams, a closed-cell cross-linked ethylene copolymer foam such as one of the Evazote^{®} line of foams, or a closed-cell, cross-linked ethylene copolymer foam such as one of the Supazote^{®} line of foams, all available from Zotefoams Plc, of London, England. In a particular example embodiment, the manifold layer 205 may comprise a closed-cell cross-linked ethylene copolymer foam as Evazote^{®} EV50.

In some embodiments, an expanded foam may be advantageously employed as the manifold layer 205. For example, the particular process by which the expanded foam is made allows additives and modifiers, for example, antimicrobial materials and/or SAPs, to be incorporated into and made integral to the polymeric material that forms the expanded foam. Efforts to likewise incorporate such additional materials into conventional foams, such as those created by chemical reaction, have proven unsuccessful, for example, due to the nature of the processes by which such conventional foams are made. As such, antimicrobials and SAPs, for example, have not successfully been likewise incorporated into such conventional foams and, instead, have been applied or coated onto the surfaces of such conventional foams. As such, the incorporation of antimicrobial materials or SAPs into an expanded foam may provide unique and beneficial properties not associated with conventional foams. Additionally, the use of an expanded foam as the manifold layer 205 may also decrease the opportunity for any in-growth of tissue into the foam during healing of a wound or other tissue site.

The fluid management layer 210 may comprise or provide a means for controlling or managing fluid flow. In some embodiments, the fluid management layer 210 may comprise or be formed from a liquid-impermeable, elastomeric material. For example, the fluid management layer 210 may comprise or be formed from a polymeric film. The fluid management layer 210 may also have a smooth or matte surface texture in some embodiments. A glossy or shiny finish better or equal to a grade B3 according to the SPI (Society of the Plastics Industry) standards may be particularly advantageous for some applications. In some embodiments, variations in surface height may be limited to acceptable tolerances. For example, the surface of the fluid management layer 210 may have a substantially flat surface, with height variations limited to 0.2 millimeters over a centimeter.

In some embodiments, the fluid management layer 210 may be hydrophobic. The hydrophobicity of the fluid management layer 210 may vary, but may have a contact angle with water of at least ninety degrees in some embodiments. In some embodiments, the fluid management layer 210 may have a contact angle with water of no more than 150 degrees. For example, in some embodiments, the contact angle of the fluid management layer 210 may be in a range of at least 90 degrees to about 120 degrees, or in a range of at least 120 degrees to 150 degrees. Water contact angles can be measured using any standard apparatus. Although manual goniometers can be used to visually approximate contact angles, contact angle measuring instruments can often include an integrated system involving a level stage, liquid dropper such as a syringe, camera, and software designed to calculate contact angles more accurately and precisely, among other things. Non-limiting examples of such integrated systems may include the FTÅ125, FTÅ200, FTÅ2000, and FTÅ4000 systems, all commercially available from First Ten Angstroms, Inc., of Portsmouth, VA, and the DTA25, DTA30, and DTA100 systems, all commercially available from Kruss GmbH of Hamburg, Germany. Unless otherwise specified, water contact angles herein are measured using deionized and distilled water on a level sample surface for a sessile drop added from a height of no more than 5 cm in air at 20-25°C and 20-50% relative humidity. Contact angles reported herein represent averages of 5-9 measured values, discarding both the highest and lowest measured values. The hydrophobicity of the fluid management layer 210 may be further enhanced with a hydrophobic coating of other materials, such as silicones and fluorocarbons, either as coated from a liquid, or plasma coated.

The fluid management layer 210 may also be suitable for welding to other layers, including the manifold layer 205. For example, the fluid management layer 210 may be adapted for welding to polyurethane foams using heat, radio frequency (RF) welding, or other methods to generate heat such as ultrasonic welding. RF welding may be particularly suitable for more polar materials, such as polyurethane, polyamides, polyesters and acrylates. Sacrificial polar interfaces may be used to facilitate RF welding of less polar film materials, such as polyethylene.

The area density of the fluid management layer 210 may vary according to a prescribed therapy or application. In some embodiments, an area density of less than 40 grams per square meter may be suitable, and an area density of about 20-30 grams per square meter may be particularly advantageous for some applications.

In some embodiments, for example, the fluid management layer 210 may comprise or be formed from a hydrophobic polymer, such as a polyethylene film. The simple and inert structure of polyethylene can provide a surface that interacts little, if any, with biological tissues and fluids, providing a surface that may encourage the free flow of liquids and low adherence, which can be particularly advantageous for many applications. More polar films suitable for laminating to a polyethylene film include polyamide, co-polyesters, ionomers, and acrylics. To aid in the bond between a polyethylene and polar film, tie layers may be used, such as ethylene vinyl acetate, or modified polyurethanes. An ethyl methyl acrylate (EMA) film may also have suitable hydrophobic and welding properties for some configurations.

As illustrated in the example of Figure 2, the fluid management layer 210 may have one or more fluid restrictions 220, which can be distributed uniformly or randomly across the fluid management layer 210. The fluid restrictions 220 may be characterized bi-directional and pressureresponsive. For example, the fluid restrictions 220 can generally comprise an elastic passage that is normally unstrained to substantially reduce liquid flow, and can expand in response to a pressure gradient or deformation of the fluid management layer 210. In some embodiments, the fluid restrictions 220 may comprise perforations in the fluid management layer 210. Perforations may be formed by removing material from the fluid management layer 210. For example, perforations may be formed by cutting through the fluid management layer 210, which may also deform the edges of the perforations in some embodiments. In the absence of a pressure gradient across the perforations or deformation of the fluid management layer 210, the passages may be sufficiently small to form a seal or flow restriction, which can substantially reduce or prevent liquid flow. Additionally or alternatively, one or more of the fluid restrictions 220 may be an elastomeric valve that is normally closed when unstrained to substantially prevent liquid flow, and can open in response to a pressure gradient or deformation of the fluid management layer 210. A fenestration in the fluid management layer 210 may be a suitable valve for some applications. Fenestrations may also be formed by removing material from the fluid management layer 210, but the amount of material removed and the resulting dimensions of the fenestrations may be an order of magnitude less than perforations, and may not deform the edges.

For example, some embodiments of the fluid restrictions 220 may comprise one or more slots or combinations of slots in the fluid management layer 210. In some examples, the fluid restrictions 220 may comprise linear slots having a length less than 4 millimeters and a width less than 1 millimeter. The length may be at least 2 millimeters, and the width may be at least 0.4 millimeters in some embodiments. A length of about 3 millimeters and a width of about 0.8 millimeter may be particularly suitable for many applications. A tolerance of about 0.1 millimeter may also be acceptable. Such dimensions and tolerances may be achieved with a laser cutter, for example. Slots of such configurations may function as imperfect valves that substantially reduce liquid flow in a normally closed or resting state. For example, such slots may form a flow restriction without being completely closed or sealed. The slots can expand or open wider in response to a pressure gradient or deformation of the fluid management layer 210 to allow increased liquid flow.

In some embodiments, the fluid restrictions 220 may be distributed across the fluid management layer 210 such that, when the fluid management layer 210 is positioned with respect to the manifold layer 205, the fluid restrictions 220 will be aligned with, overlap, in registration with, or otherwise fluidly coupled to the collapsible apertures 310, the fluid apertures 320, the channels 610, or combinations thereof.

The contact layer 215 may comprise a sealing layer comprising or formed from a soft, pliable material suitable for providing a fluid seal with a tissue site, and may have a substantially flat surface. For example, the contact layer 215 may comprise, without limitation, a silicone gel, a soft silicone, hydrocolloid, hydrogel, polyurethane gel, polyolefin gel, hydrogenated styrenic copolymer gel, a foamed gel, a soft closed-cell foam such as polyurethanes and polyolefins coated with an adhesive, polyurethane, polyolefin, or hydrogenated styrenic copolymers. In some embodiments, the contact layer 215 may have a thickness between about 200 microns (µm) and about 1000 microns (µm). In some embodiments, the contact layer 215 may have a hardness between about 5 Shore OO and about 80 Shore OO. Further, the contact layer 215 may be comprised of hydrophobic or hydrophilic materials.

In some embodiments, the contact layer 215 may be a hydrophobic-coated material. For example, the contact layer 215 may be formed by coating a spaced material, such as, for example, woven, nonwoven, molded, or extruded mesh with a hydrophobic material. The hydrophobic material for the coating may be a soft silicone, for example.

The contact layer 215 may have a periphery 225 surrounding or around an interior portion 230, and apertures 235 disposed through the periphery 225 and the interior portion 230. The interior portion 230 may correspond to a surface area of the manifold layer 205 in some examples. The contact layer 215 may also have corners 240 and edges 245. The corners 240 and the edges 245 may be part of the periphery 225. The contact layer 215 may have an interior border 250 around the interior portion 230, disposed between the interior portion 230 and the periphery 225. The interior border 250 may be substantially free of the apertures 235, as illustrated in the example of Figure 2. In some examples, as illustrated in Figure 2, the interior portion 230 may be symmetrical and centrally disposed in the contact layer 215.

The apertures 235 may be formed by cutting or by application of local RF or ultrasonic energy, for example, or by other suitable techniques for forming an opening. The apertures 235 may have a uniform distribution pattern, or may be randomly distributed on the contact layer 215. The apertures 235 in the contact layer 215 may have many shapes, including circles, squares, stars, ovals, polygons, slits, complex curves, rectilinear shapes, triangles, for example, or may have some combination of such shapes.

Each of the apertures 235 may have uniform or similar geometric properties. For example, in some embodiments, each of the apertures 235 may be circular apertures, having substantially the same diameter. In some embodiments, the diameter of each of the apertures 235 may be from about 1 millimeter to about 50 millimeters. In other embodiments, the diameter of each of the apertures 235 may be from about 1 millimeter to about 20 millimeters.

In other embodiments, geometric properties of the apertures 235 may vary. For example, the diameter of the apertures 235 may vary depending on the position of the apertures 235 in the contact layer 215, as illustrated in Figure 2. In some embodiments, the diameter of the apertures 235 in the periphery 225 of the contact layer 215 may be larger than the diameter of the apertures 235 in the interior portion 230 of the contact layer 215. For example, in some embodiments, the apertures 235 disposed in the periphery 225 may have a diameter between about 9.8 millimeters to about 10.2 millimeters. In some embodiments, the apertures 235 disposed in the corners 240 may have a diameter between about 7.75 millimeters to about 8.75 millimeters. In some embodiments, the apertures 235 disposed in the interior portion 230 may have a diameter between about 1.8 millimeters to about 2.2 millimeters.

At least one of the apertures 235 in the periphery 225 of the contact layer 215 may be positioned at the edges 245 of the periphery 225, and may have an interior cut open or exposed at the edges 245 that is in fluid communication in a lateral direction with the edges 245. The lateral direction may refer to a direction toward the edges 245 and in the same plane as the contact layer 215. As shown in the example of Figure 2, the apertures 235 in the periphery 225 may be positioned proximate to or at the edges 245 and in fluid communication in a lateral direction with the edges 245. The apertures 235 positioned proximate to or at the edges 245 may be spaced substantially equidistant around the periphery 225 as shown in the example of Figure 2. Alternatively, the spacing of the apertures 235 proximate to or at the edges 245 may be irregular.

In the example of Figure 2, the dressing 110 may further include an attachment device, such as an adhesive 255. The adhesive 255 may be, for example, a medically-acceptable, pressure-sensitive adhesive that extends about a periphery, a portion, or the entire cover 125. In some embodiments, for example, the adhesive 255 may comprise an acrylic adhesive having a coating weight between 25-65 grams per square meter (g.s.m.). Additionally or alternatively, in some embodiments the adhesive 255 may comprise a silicone-based adhesive. Thicker adhesives, or combinations of adhesives, may be applied in some embodiments to improve the seal and reduce leaks. The adhesive 255 may be a layer having substantially the same shape as the periphery 225. In some embodiments, such a layer of the adhesive 255 may be continuous or discontinuous. Discontinuities in the adhesive 255 may be provided by apertures or holes (not shown) in the adhesive 255. The apertures or holes in the adhesive 255 may be formed after application of the adhesive 255 or by coating the adhesive 255 in patterns on a carrier layer, such as, for example, a side of the cover 125. Apertures or holes in the adhesive 255 may also be sized to enhance the MVTR of the dressing 110 in some example embodiments.

As illustrated in the example of Figure 2, in some embodiments, a release liner 260 may be attached to or positioned adjacent to the contact layer 215, for example, to protect the adhesive 255 prior to use. The release liner 260 may also provide stiffness, such as to assist with deployment of the dressing 110. The release liner 260 may be, for example, a casting paper, a film, or polyethylene. Further, in some embodiments, the release liner 260 may be a polyester material such as polyethylene terephthalate (PET), or a similar polar semi-crystalline polymer. The use of a polar semi-crystalline polymer for the release liner 260 may substantially preclude wrinkling or other deformation of the dressing 110. For example, the polar semi-crystalline polymer may be highly orientated and resistant to softening, swelling, or other deformation that may occur when brought into contact with components of the dressing 110, or when subjected to temperature or environmental variations, or sterilization. In some embodiments, the release liner 260 may have a surface texture that may be imprinted on an adjacent layer, such as the contact layer 215. Further, a release agent may be disposed on a side of the release liner 260 that is configured to contact the contact layer 215. For example, the release agent may be a silicone coating and may have a release factor suitable to facilitate removal of the release liner 260 by hand and without damaging or deforming the dressing 110. In some embodiments, the release agent may be a fluorocarbon or a fluorosilicone, for example. In other embodiments, the release liner 260 may be uncoated or otherwise used without a release agent.

Figure 2 also illustrates one example of a fluid conductor 265 and a dressing interface 270. As shown in the example of Figure 2, the fluid conductor 265 may be a flexible tube, which can be fluidly coupled on one end to the dressing interface 270. The dressing interface 270 may be an elbow connector, as shown in the example of Figure 2, which can be placed over an aperture 275 in the cover 125 to provide a fluid path between the fluid conductor 265 and the tissue interface 120.

In some embodiments, for example, in the example of Figure 2, the cover 125 and the contact layer 215 may be sized such that a peripheral portion of the cover 125 and the periphery 225 of the contact layer 215 each extend beyond the perimeter of the manifold layer 205 and the fluid management layer 210. For example, the cover 125 and the contact layer 215 may have dimensions such that a perimeter of the cover 125 is substantially coextensive with the edges 245 of the periphery 225 of the contact layer 215 when the cover 125, the manifold layer 205, the fluid management layer 210, and the contact layer 215 are positioned with respect to each other. In some embodiments, the contact layer 215 and the cover 125 may be coupled, such as via the adhesive 255, to enclose the manifold layer 205 and the fluid management layer 210, also allowing a portion of the adhesive 255 to be exposed through the apertures 235.

Alternatively, in some embodiments, the cover 125, the manifold layer 205, the fluid management layer 210, and the contact layer 215 may have substantially equivalent sizes and shapes, for example, such that each of the cover 125, the manifold layer 205, the fluid management layer 210, and the contact layer 215 are coextensive with respect to outline or perimeter when the cover 125, the manifold layer 205, the fluid management layer 210, and the contact layer 215 are positioned with respect to each other. Also in some embodiments, each of the cover 125, the manifold layer 205, the fluid management layer 210, and the contact layer 215 may be attached, such as via an adhesive or RF welding, to an immediately-adjacent layer.

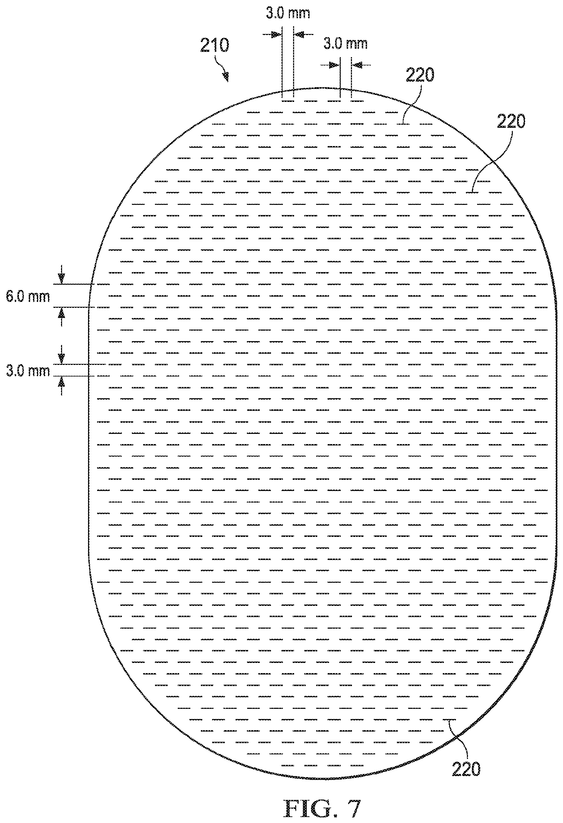

Figure 7 is a schematic view of an example of the fluid management layer 210, illustrating additional details that may be associated with some embodiments. As illustrated in the example of Figure 7, the fluid restrictions 220 may each consist essentially of one or more linear slots having a length of about 3 millimeters. Figure 7 additionally illustrates an example of a uniform distribution pattern of the fluid restrictions 220. In Figure 7, the fluid restrictions 220 are substantially coextensive with the fluid management layer 210, and are distributed across the fluid management layer 210 in a grid of parallel rows and columns, in which the slots are also mutually parallel to each other. In some embodiments, the rows may be spaced about 3 millimeters on center, and the fluid restrictions 220 within each of the rows may be spaced about 3 millimeters on center as illustrated in the example of Figure 7. The fluid restrictions 220 in adjacent rows may be aligned or offset. For example, adjacent rows may be offset, as illustrated in Figure 7, so that the fluid restrictions 220 are aligned in alternating rows and separated by about 6 millimeters. The spacing of the fluid restrictions 220 may vary in some embodiments to increase the density of the fluid restrictions 220 according to therapeutic requirements.

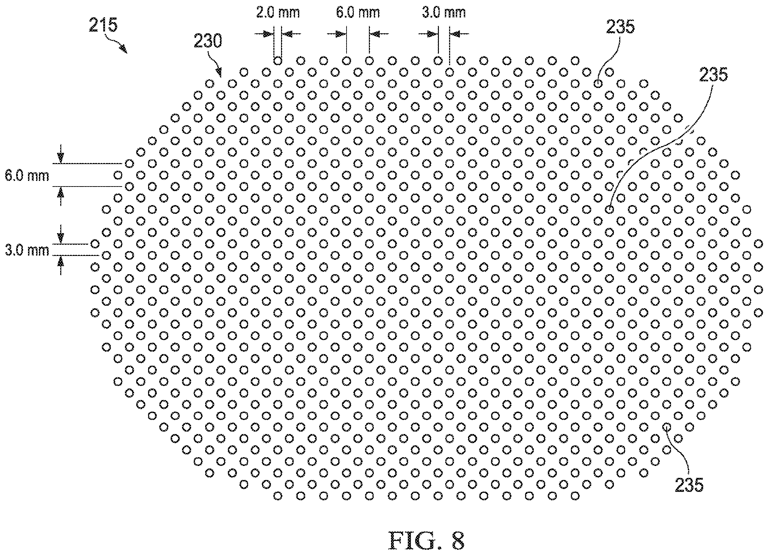

Figure 8 is a schematic view of an example configuration of the apertures 235, illustrating additional details that may be associated with some embodiments of the contact layer 215. In some embodiments, the apertures 235 illustrated in Figure 8 may be associated only with the interior portion 230. In the example of Figure 8, the apertures 235 are generally circular and have a diameter of about 2 millimeters. Figure 8 also illustrates an example of a uniform distribution pattern of the apertures 235 in the interior portion 230. In Figure 8, the apertures 235 are distributed across the interior portion 230 in a grid of parallel rows and columns. Within each row and column, the apertures 235 may be equidistant from each other, as illustrated in the example of Figure 8. Figure 8 illustrates one example configuration that may be particularly suitable for many applications, in which the apertures 235 are spaced about 6 millimeters apart along each row and column, with a 3 millimeter offset.

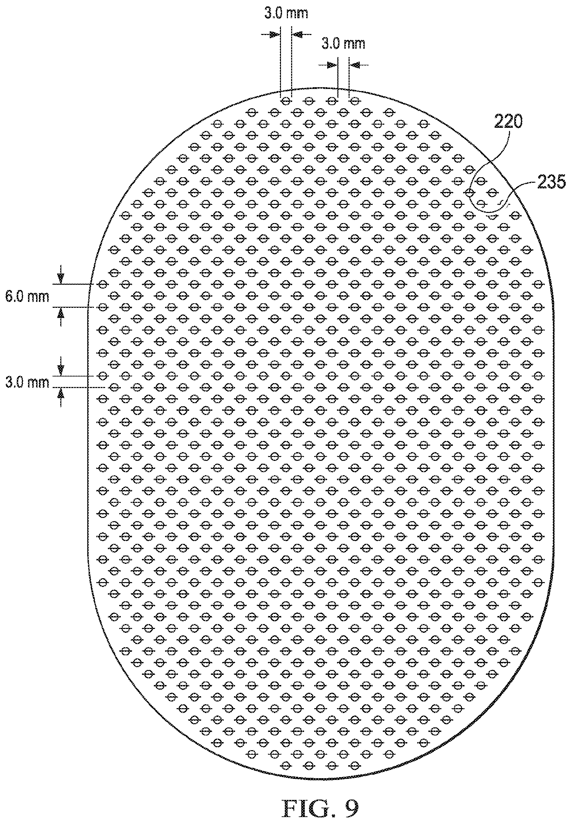

Figure 9 is a schematic view of the example contact layer 215 of Figure 8 overlaid on the fluid management layer 210 of Figure 7, illustrating additional details that may be associated with some example embodiments of the tissue interface 120. For example, as illustrated in Figure 9, the fluid restrictions 220 may be aligned, overlapping, in registration with, or otherwise fluidly coupled to the apertures 235 in some embodiments. In some embodiments, one or more of the fluid restrictions 220 may be registered with the apertures 235 only in the interior portion 230, or only partially registered with the apertures 235. The fluid restrictions 220 in the example of Figure 9 are generally configured so that each of the fluid restrictions 220 is registered with only one of the apertures 235. In other examples, one or more of the fluid restrictions 220 may be registered with more than one of the apertures 235. For example, any one or more of the fluid restrictions 220 may be a perforation or a fenestration that extends across two or more of the apertures 235. Additionally or alternatively, one or more of the fluid restrictions 220 may not be registered with any of the apertures 235.

As illustrated in the example of Figure 9, the apertures 235 may be sized to expose a portion of the fluid management layer 210, the fluid restrictions 220, or both through the contact layer 215. In some embodiments, each of the apertures 235 may be sized to expose no more than two of the fluid restrictions 220. In some examples, the length of each of the fluid restrictions 220 may be substantially equal to or less than the diameter of each of the apertures 235. In some embodiments, the average dimensions of the fluid restrictions 220 are substantially similar to the average dimensions of the apertures 235. For example, the apertures 235 may be elliptical in some embodiments, and the length of each of the fluid restrictions 220 may be substantially equal to the major axis or the minor axis. In some embodiments, though, the dimensions of the fluid restrictions 220 may exceed the dimensions of the apertures 235, and the size of the apertures 235 may limit the effective size of the fluid restrictions 220 exposed to the lower surface of the dressing 110.

One or more of the components of the dressing 110 may additionally be treated with an antimicrobial agent in some embodiments. For example, the manifold layer 205 may be a foam, mesh, or non-woven coated with an antimicrobial agent. In some embodiments, the manifold layer 205 may comprise antimicrobial elements, such as fibers coated with an antimicrobial agent. Additionally or alternatively, some embodiments of the fluid management layer 210 may be a polymer coated or mixed with an antimicrobial agent. In other examples, the fluid conductor 265 may additionally or alternatively be treated with one or more antimicrobial agents. Suitable antimicrobial agents may include, for example, metallic silver, PHMB, iodine or its complexes and mixes such as povidone iodine, copper metal compounds, chlorhexidine, or some combination of these materials.

Individual components of the dressing 110 may be bonded or otherwise secured to one another with a solvent or non-solvent adhesive, or with thermal welding, for example, without adversely affecting fluid management. Further, the fluid management layer 210 or the manifold layer 205 may be coupled to the interior border 250 of the contact layer 215 in any suitable manner, such as with a weld or an adhesive, for example.

The cover 125, the manifold layer 205, the fluid management layer 210, the contact layer 215, or various combinations may be assembled before application or *in situ.* For example, the cover 125 may be laminated to the manifold layer 205, and the fluid management layer 210 may be laminated to the manifold layer 205 opposite the cover 125 in some embodiments. The contact layer 215 may also be coupled to the fluid management layer 210 opposite the manifold layer 205 in some embodiments. In some embodiments, two or more layers of the tissue interface 120 may be coextensive. For example, the manifold layer 205 may be coextensive with the fluid management layer 210, as illustrated in the embodiment of Figure 2. In some embodiments, the dressing 110 may be provided as a single, composite dressing. For example, the contact layer 215 may be coupled to the cover 125 to enclose the manifold layer 205 and the fluid management layer 210, wherein the contact layer 215 is configured to face a tissue site.

In use, the release liner 260 (if included) may be removed to expose the contact layer 215, which may be placed within, over, on, or otherwise proximate to a tissue site, particularly a surface tissue site and adjacent epidermis. The contact layer 215 and the fluid management layer 210 may be interposed between the manifold layer 205 and the tissue site, which can substantially reduce or eliminate adverse interaction with the manifold layer 205. For example, the contact layer 215 may be placed over a surface wound (including edges of the wound) and undamaged epidermis to prevent direct contact with the manifold layer 205. Treatment of a surface wound or placement of the dressing 110 on a surface wound includes placing the dressing 110 immediately adjacent to the surface of the body or extending over at least a portion of the surface of the body. Treatment of a surface wound does not include placing the dressing 110 wholly within the body or wholly under the surface of the body, such as placing a dressing within an abdominal cavity. In some applications, the interior portion 230 of the contact layer 215 may be positioned adjacent to, proximate to, or covering a tissue site. In some applications, at least some portion of the fluid management layer 210, the fluid restrictions 220, or both may be exposed to a tissue site through the contact layer 215. The periphery 225 of the contact layer 215 may be positioned adjacent to or proximate to tissue around or surrounding the tissue site. The contact layer 215 may be sufficiently tacky to hold the dressing 110 in position, while also allowing the dressing 110 to be removed or re-positioned without trauma to the tissue site.

Removing the release liner 260 can also expose the adhesive 255, and the cover 125 may be attached to an attachment surface. For example, the cover 125 may be attached to epidermis peripheral to a tissue site, around the manifold layer 205 and the fluid management layer 210. The adhesive 255 may be in fluid communication with an attachment surface through the apertures 235 in at least the periphery 225 of the contact layer 215 in some embodiments. The adhesive 255 may also be in fluid communication with the edges 245 through the apertures 235 exposed at the edges 245.

Once the dressing 110 is in the desired position, the adhesive 255 may be pressed through the apertures 235 to bond the dressing 110 to the attachment surface. The apertures 235 at the edges 245 may permit the adhesive 255 to flow around the edges 245 for enhancing the adhesion of the edges 245 to an attachment surface.

In some embodiments, the bond strength of the adhesive 255 may vary in different locations of the dressing 110. For example, the adhesive 255 may have a lower bond strength in locations adjacent to the contact layer 215 where the apertures 235 are relatively larger, and may have a higher bond strength where the apertures 235 are smaller. Adhesive 255 with lower bond strength in combination with larger apertures 235 may provide a bond comparable to the adhesive 255 with higher bond strength in locations having smaller apertures 235.

The geometry and dimensions of the tissue interface 120, the cover 125, or both may vary to suit a particular application or anatomy. For example, the geometry or dimensions of the tissue interface 120 and the cover 125 may be adapted to provide an effective and reliable seal against challenging anatomical surfaces, such as an elbow or heel, at and around a tissue site. Additionally or alternatively, the dimensions may be modified to increase the surface area for the contact layer 215 to enhance the movement and proliferation of epithelial cells at a tissue site and reduce the likelihood of granulation tissue in-growth.

Further, the dressing 110 may permit re-application or re-positioning to reduce or eliminate leaks, which can be caused by creases and other discontinuities in the dressing 110 and a tissue site. The ability to rectify leaks may increase the reliability of the therapy and reduce power consumption in some embodiments.

Additionally or alternatively, in some embodiments, such as where all components of the tissue interface 120 have a common perimeter or outline (for example, in an embodiment where the cover 125, the manifold layer 205, the fluid management layer 210, and the contact layer 215 are coextensive with respect to outline or perimeter), an attachment device can be disposed around edges of the cover 125. An adhesive disposed on the attachment device may pressed onto the cover 125 and the epidermis peripheral to a tissue site (or other attachment surface) to fix the dressing 110 in position and to seal the exposed perimeter of the tissue interface 120.

Thus, the dressing 110 in the example of Figure 2 can provide a sealed therapeutic environment proximate to a tissue site, substantially isolated from the external environment, and the negative-pressure source 105 can reduce the pressure in the sealed therapeutic environment. The contact layer 215 may provide an effective and reliable seal against challenging anatomical surfaces, such as an elbow or heel, at and around a tissue site. Further, the dressing 110 may permit re-application or re-positioning, to correct air leaks caused by creases and other discontinuities in the dressing 110, for example. The ability to rectify leaks may increase the efficacy of the therapy and reduce power consumption in some embodiments.

If not already configured, the dressing interface 270 may be disposed over the aperture 275 and attached to the cover 125. The fluid conductor 265 may be fluidly coupled to the dressing interface 270 and to the negative-pressure source 105.

In some embodiments, negative pressure applied through the tissue interface 120 may be applied to the tissue interface 120. The negative pressure may be effective to cause the manifold layer 205 to exhibit radial collapse such that the area of the first surface 206 and/or the second surface 207, when subjected to negative pressure, is decreased relative to the area of the respective surface when the manifold layer 205 is not subjected to negative pressure. For example, the radial collapse may be substantially due to collapse of one or more of the collapsible apertures 310. While the collapsible apertures 310 may collapse when subjected to negative pressure, the fluid apertures 320 may remain substantially un-deformed, for example, such that fluids may be communicated between the first surface 206 and the second surface 207 via the fluid apertures 320.

In some embodiments, the radial collapse exhibited by the manifold layer 205 may have the effect of drawing the edges of the tissue site (e.g., a wound) together, for example, toward the center, which may help to reduce edema and/or to apply load to the edges of the wound. The radial collapse resulting from the collapse of the collapsible apertures 310 may be particularly beneficial in the context of a closed-cell foam or, alternatively, a felted foam, for example, because such foams may not have the capacity to collapse radially as a result of the collapse of the internal pores as with conventional, unfelted, open-cell foams. With closed-cell foams, the pores are not evacuated under negative pressure and cannot collapse to cause radial collapse of the manifold layer 205. As such, the collapsible apertures 310 may allow a closed-cell foams to exhibit radial collapse while nonetheless imparting additional advantages such as imperviousness to in-growth and/or incorporation of additives such as antimicrobials and SAPs.

If the negative-pressure source 105 is removed or turned-off, the pressure differential across the fluid restrictions 220 the negative pressure applied through the tissue interface 120 may dissipate, allowing the collapsible apertures 310 to return to an uncollapsed or resting state and, likewise, allowing the manifold layer 205 to return to an uncollapsed or resting state.

Additionally, negative pressure applied through one or more layers of the tissue interface 120 may create a negative pressure differential across the fluid restrictions 220 in the fluid management layer 210, which may open or expand the fluid restrictions 220 from their resting state. For example, in some embodiments in which the fluid restrictions 220 may comprise substantially closed fenestrations through the fluid management layer 210, a pressure gradient across the fenestrations or deformation of the fluid management layer 210 can strain the adjacent material of the fluid management layer 210 and increase the dimensions of the fenestrations to allow liquid movement through them, similar to the operation of a duckbill valve. Opening the fluid restrictions 220 can allow exudate and other liquid movement through the fluid restrictions 220 into the manifold layer 205 and the container 115. Changes in pressure can also cause the manifold layer 205 to expand and contract, and the interior border 250 may protect the epidermis from irritation. The fluid management layer 210 and the contact layer 215 can also substantially reduce or prevent exposure of tissue to the manifold layer 205, which can inhibit growth of tissue into the manifold layer 205.

Also if the negative-pressure source 105 is removed or turned-off, the pressure differential across the fluid restrictions 220 may dissipate, allowing the fluid restrictions 220 to return to their resting state and prevent or reduce the rate at which exudate or other liquid from returning to the tissue site through the fluid management layer 210.

In some applications, a filler may also be disposed between a tissue site and the contact layer 215. For example, if the tissue site is a surface wound, a wound filler may be applied interior to the periwound, and the contact layer 215 may be disposed over the periwound and the wound filler. In some embodiments, the filler may comprise a manifold, such as an open-cell foam. The filler may comprise or be formed from the same material as the manifold layer 205 in some embodiments.

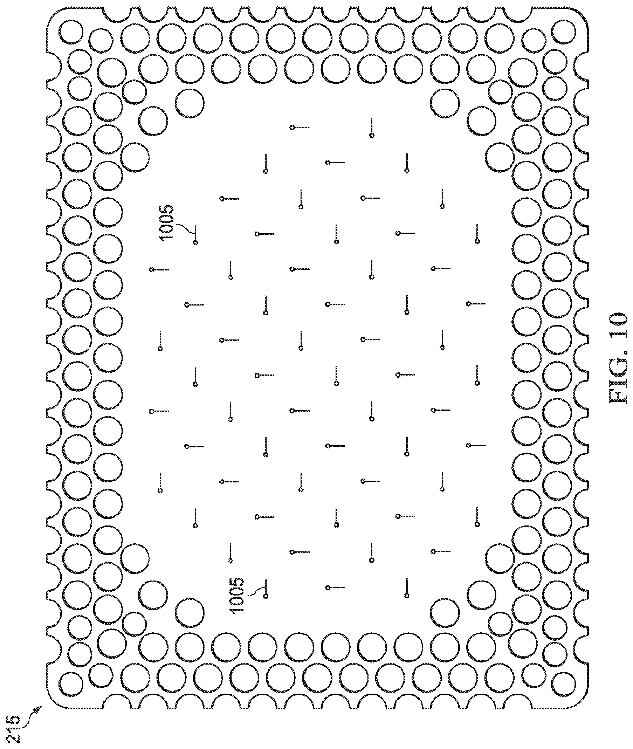

Figure 10 is a schematic view of another example of the contact layer 215, illustrating additional details that may be associated with some embodiments. As shown in the example of Figure 10, the contact layer 215 may have one or more fluid restrictions, such as valves 1005, instead of or in addition to the apertures 235 in the interior portion 230. Moreover, the valves 1005 may be included in the contact layer 215 in addition to or instead of the fluid restrictions 220 in the fluid management layer 210. In some embodiments in which the contact layer 215 includes one or more of the valves 1005, the fluid management layer 210 may be omitted. For example, in some embodiments, the tissue interface 120 may consist essentially of the manifold layer 205 and the contact layer 215 of Figure 10 with the valves 1005 disposed in the interior portion 230.

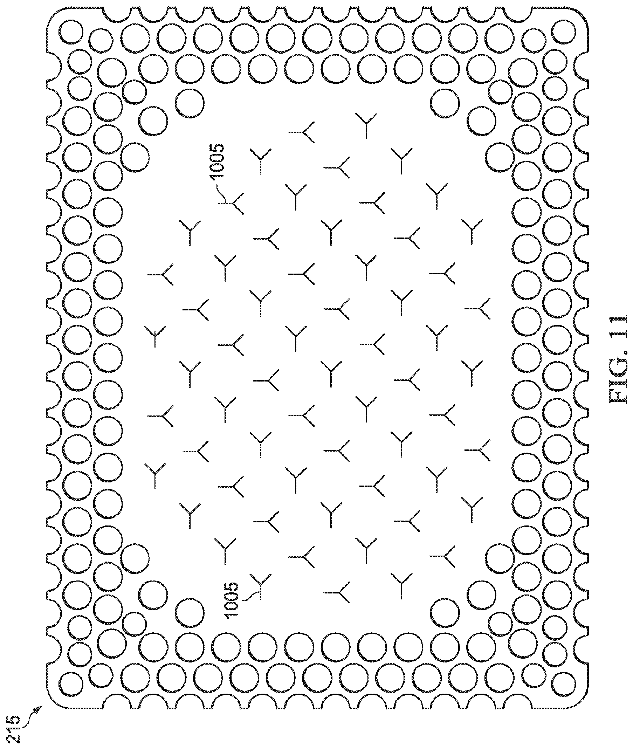

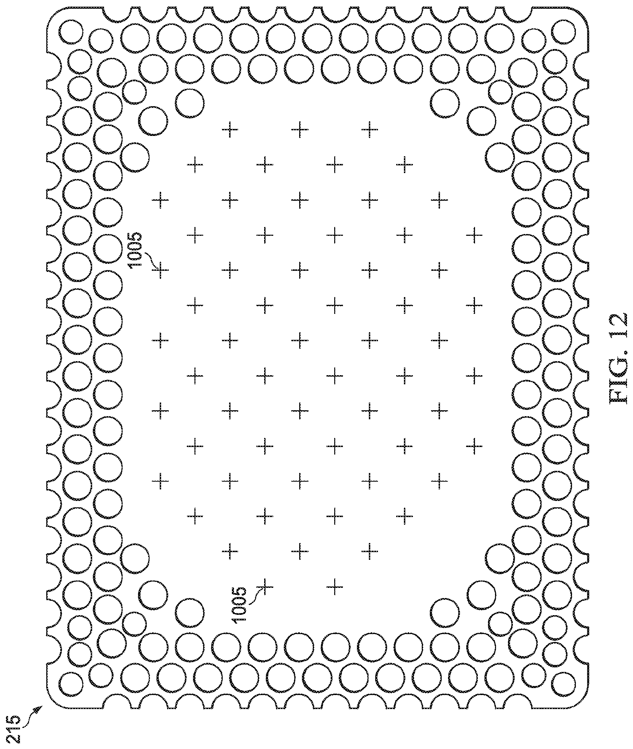

Figure 11 and Figure 12 illustrate other example configurations of the valves 1005, in which the valves 1005 each generally comprise a combination of intersecting slits or cross-slits.

Methods of treating a surface wound to promote healing and tissue granulation may include applying the dressing 110 to a surface wound and sealing the dressing 110 to epidermis adjacent to the surface wound. For example, the contact layer 215 may be placed over the surface wound, covering at least a portion of the edge of the surface wound and a periwound adjacent to the surface wound. The cover 125 may also be attached to epidermis around the contact layer 215. The dressing 110 may be fluidly coupled to a negative-pressure source, such as the negative-pressure source 105. Negative pressure from the negative-pressure source 105 may be applied to the dressing 110, opening the fluid restrictions 220. The fluid restrictions 220 can be closed by blocking, stopping, or reducing the negative pressure. The fluid management layer 210 and the contact layer 215 can substantially prevent exposure of tissue in the surface wound to the manifold layer 205, inhibiting growth of tissue into the manifold layer 205. The dressing 110 can also substantially prevent maceration of the periwound.

The systems, apparatuses, and methods described herein may provide significant advantages over prior dressings. For example, some dressings for negative-pressure therapy can require time and skill to be properly sized and applied to achieve a good fit and seal. In contrast, some embodiments of the dressing 110 provide a negative-pressure dressing that is simple to apply, reducing the time to apply and remove. In some embodiments, for example, the dressing 110 may be a fullyintegrated negative-pressure therapy dressing that can be applied to a tissue site (including on the periwound) in one step, without being cut to size, while still providing or improving many benefits of other negative-pressure therapy dressings that require sizing. Such benefits may include good manifolding, beneficial granulation, protection of the peripheral tissue from maceration, and a lowtrauma and high-seal bond. These characteristics may be particularly advantageous for surface wounds having moderate depth and medium-to-high levels of exudate. Some embodiments of the dressing 110 may remain on the tissue site for at least 5 days, and some embodiments may remain for at least 7 days. Antimicrobial agents in the dressing 110 may extend the usable life of the dressing 110 by reducing or eliminating infection risks that may be associated with extended use, particularly use with infected or highly exuding wounds.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context.

Features, elements, and aspects described in the context of some embodiments may also be omitted, combined, or replaced by alternative features serving the same, equivalent, or similar purpose without departing from the scope of the invention defined by the appended claims. For example, one or more of the features of some layers may be combined with features of other layers to provide an equivalent function.

Components may also be combined or eliminated in various configurations for purposes of sale, manufacture, assembly, or use. For example, in some configurations the dressing 110, the container 115, or both may be eliminated or separated from other components for manufacture or sale. In other example configurations, the controller 130 may also be manufactured, configured, assembled, or sold independently of other components.

The appended claims set forth novel and inventive aspects of the subject matter described above. Certain features, elements, or aspects may be omitted from the claims if not necessary to distinguish the novel and inventive features from what is already known to a person having ordinary skill in the art.

Aspects of the invention are disclosed in the following numbered clauses:
1. A dressing for treating a tissue site with negative pressure, the dressing comprising:
   a fluid management layer comprising a polymer film having a plurality of fluid restrictions extending through the polymer film and configured to deform; and
   a manifold layer coupled to the fluid management layer, the manifold layer having a first surface facing the fluid management layer, a second surface opposite the first surface, and a thickness extending between the first surface and the second surface, the manifold layer comprising a plurality of collapsible apertures extending at least partially through the thickness of the manifold layer from the first surface, the collapsible apertures configured to deform in response to a negative pressure applied to the manifold layer so that the an area of the first surface, the second surface, or both, when subjected to negative pressure, is decreased relative to the area of the first surface or the second surface when the manifold layer is not subjected to negative pressure.
2. The dressing of clause 1, wherein each of the plurality of collapsible apertures comprises:
   a length parallel to the first surface of the manifold layer; and
   a width parallel to the first surface of the manifold layer and perpendicular to the length,
   wherein the length of each of the plurality of collapsible aperture is greater than its width.
3. The dressing of clause 2, wherein the length is at least 1.5 times the width.
4. The dressing of one of clauses 2-3, wherein the length and the width of each of the collapsible apertures is substantially constant.
5. The dressing of one of clauses 1-4, wherein each of the plurality of collapsible apertures are rectangular, elongate, diamond-shaped, square, oval, ovoid, irregular, polygonal, or amorphous.
6. The dressing of one of clauses 1-5, wherein the plurality of collapsible apertures includes a first portion of collapsible apertures having a first orientation and a second portion of collapsible apertures having a second orientation.
7. The dressing of clause 6, wherein the first orientation deviates from the second orientation by between 75 degrees and 105 degrees.
8. The dressing of one of clauses 6-7, wherein the first orientation is substantially perpendicular to the second orientation.
9. The dressing of one of clauses 6-8, wherein the first portion of collapsible apertures is arranged in a first plurality of rows and the second portion of collapsible apertures is arranged in a second plurality of rows.
10. The dressing of clause 9, wherein each of the first plurality of rows is adjacent to one of the second plurality of rows.
11. The dressing of one of clauses 6-10, wherein a perpendicular bisector of the length of one of the first portion of collapsible apertures extends through at least one of the second portion of collapsible apertures.
12. The dressing of one of clauses 6-11, wherein a clauses dicular bisector of the length of one of the first portion of collapsible apertures extends through a mid-point of the width of one of the second portion of collapsible apertures.
13. The dressing of one of clauses 1-12, wherein the manifold layer further comprises a plurality of fluid apertures extending through the surface of the manifold between the first surface and the second surface, wherein the fluid apertures are configured to exhibit little or no deformation in response to the negative pressure applied to the manifold layer relative to the plurality of collapsible apertures.
14. The dressing of one of clauses 1-13, wherein the at least one of the first surface and the second surface comprises a sculpted surface comprising a plurality of fluid channels disposed within the sculpted surface.
15. The dressing of clause 14, wherein each of the first surface and the second surface comprises a sculpted surface comprising a plurality of fluid channels disposed within the sculpted surface.
16. The dressing of one of clauses 14-15, wherein the plurality of channels includes a first portion of fluid channels and a second portion of fluid channels, wherein one of the first portion of fluid channels intersects two or more of the second portion of fluid channels.
17. The dressing of one of clauses 14-16, wherein the first portion of fluid channels and the second portion of fluid channels are perpendicular.
18. The dressing of one of clauses 1-17, wherein the manifold layer comprises an expanded foam material.
19. The dressing of clause 18, wherein the expanded foam material is formed by a process comprising:
   impregnating a polymeric material with an inert gas at high heat and pressure to form an impregnated polymeric material; and
   expanding the impregnated polymeric material to form the expanded foam material.
20. The dressing of one of clauses 18-19, wherein the expanded foam comprises an antimicrobial material incorporated within the expanded foam.
21. The dressing of one of clauses 1-20, wherein the manifold layer comprises a closed-cell foam.
22. The dressing of one of clauses 1-17, wherein the manifold layer comprises a felted foam.
23. The dressing of one of clauses 1-22, further comprising a polymer drape coupled to the manifold layer opposite the fluid management layer.
24. The dressing of one of clauses 1-23, wherein the polymer drape comprises a margin that extends beyond the manifold layer and the fluid management layer, and an adhesive layer disposed in the margin.
25. The dressing of one of clauses 1-24, further comprising a contact layer coupled to the fluid management layer opposite the manifold layer, the contact layer comprising a plurality of apertures.
26. The dressing of clause 1-25, further comprising a fluid port configured to provide a route of fluid communication between the manifold layer and a fluid conductor.
27. A dressing for treating a tissue site with negative pressure, the dressing comprising:
   a tissue interface layer comprising a polymer film having a plurality of fluid restrictions extending through the polymer film and configured to deform; and
   a manifold layer coupled to the fluid management layer, the manifold layer having a first surface facing the fluid management layer, a second surface opposite the first surface, and a thickness extending between the first surface and the second surface, the manifold comprising an expanded foam and configured to exhibit a radial collapse such that the an area of the first surface, the second surface, or both, when the manifold layer is subjected to negative pressure, is decreased relative to the area of the first surface or the second surface when the manifold layer is not subjected to negative pressure.
28. The dressing of clause 27, wherein the expanded foam material is formed from a process comprising:
   impregnation of a polymeric material with an inert gas at high heat and pressure to form an impregnated polymeric material; and
   expansion of the impregnated polymeric material to form the expanded foam material.
29. The dressing of clause 28, wherein the polymeric material comprises a cross-linked ethylene-vinyl acetate copolymer.
30. The dressing of clause 28, wherein the polymeric material comprises a cross-linked polyolefin.
31. The dressing of clause 28, wherein the polymeric material comprises a cross-linked polyethylene.
32. The dressing of clause 28, wherein the polymeric material comprises a cross-linked ethyl-methyl-acrylate copolymer.
33. The dressing of one of clauses 28-32, wherein the polymeric material comprises an antimicrobial material.
34. The dressing of one of clauses 28-33, wherein the polymeric material comprises an antimicrobial material comprises an absorbent.
35. The dressing of one of clauses 28-34, wherein the inert gas comprises at least 95% by weight of nitrogen gas.
36. The dressing of one of clauses 27-35, wherein the expanded foam comprises a cross-linked ethylene-vinyl acetate copolymer.
37. The dressing of one of clauses 27-35, wherein the expanded foam comprises a cross-linked polyolefin.
38. The dressing of one of clauses 27-35, wherein the expanded foam comprises a cross-linked polyethylene.
39. The dressing of one of clauses 27-35, wherein the expanded foam comprises a cross-linked ethyl-methyl-acrylate copolymer.
40. The dressing of one of clauses 27-39, wherein the expanded foam comprises an antimicrobial material incorporated within the expanded foam.
41. The dressing of one of clauses 27-40, wherein the expanded foam comprises a closed-cell foam.
42. The dressing of one of clauses 27-41, wherein the manifold layer further comprises a plurality of collapsible apertures extending at least partially through the thickness of the manifold layer from the first surface, the collapsible apertures configured to deform in response to a negative pressure applied to the manifold layer.
43. The dressing of clause 42, wherein each of the plurality of collapsible apertures comprises:
   a length parallel to the first surface of the manifold layer; and
   a width parallel to the first surface of the manifold layer and perpendicular to the length,
   wherein the length of each of the plurality of collapsible aperture is greater than its width.
44. The dressing of clause 43, wherein the length is at least 1.5 times the width.
45. The dressing of one of clauses 43-44, wherein the length and the width of each of the collapsible apertures is substantially constant.
46. The dressing of one of clauses 42-45, wherein each of the plurality of collapsible apertures are rectangular, elongate, diamond-shaped, square, oval, ovoid, irregular, polygonal, or amorphous.
47. The dressing of one of clauses 43-46, wherein the plurality of collapsible apertures includes a first portion of collapsible apertures having a first orientation and a second portion of collapsible apertures having a second orientation.
48. The dressing of clause 47, wherein the first orientation deviates from the second orientation by between 75 degrees and 105 degrees.
49. The dressing of one of clauses 47-48, wherein the first orientation is substantially perpendicular to the second orientation.
50. The dressing of one of clauses 47-49, wherein the first portion of collapsible apertures is arranged in a first plurality of rows and the second portion of collapsible apertures is arranged in a second plurality of rows.
51. The dressing of clause 50, wherein each of the first plurality of rows is adjacent to one of the second plurality of rows.
52. The dressing of one of clauses 47-51, wherein a perpendicular bisector of the length of one of the first portion of collapsible apertures extends through at least one of the second portion of collapsible apertures.
53. The dressing of one of clauses 47-52, wherein a perpendicular bisector of the length of one of the first portion of collapsible apertures extends through a mid-point of the width of one of the second portion of collapsible apertures.
54. The dressing of one of clauses 27-53, wherein the manifold layer further comprises a plurality of fluid apertures extending through the surface of the manifold between the first surface and the second surface, wherein the fluid apertures are configured to exhibit little or no deformation in response to a negative pressure relative to the plurality of collapsible apertures.
55. A system for treating a tissue site, the system comprising:
   the dressing of any of clauses 1-54; and
   a negative-pressure source fluidly coupled to the dressing.
56. The system of clause 55, further comprising a fluid container fluidly coupled between the dressing and the negative-pressure source.
57. A method of treating a surface wound with negative pressure, the method comprising:
   applying the dressing of any of clauses 1-54 to the surface wound;
   sealing the dressing to epidermis adjacent to the surface wound;
   fluidly coupling the dressing to a negative-pressure source; and
   applying negative pressure from the negative-pressure source to the dressing.
58. The method of clause 57, wherein the manifold layer is not substantially exposed to the tissue site during the step of applying negative pressure.
59. The method of any one of clauses 57-58, wherein applying the dressing comprises disposing at least part of the dressing across an edge of the surface wound.
60. The method of any one of clauses 57-59, wherein applying negative pressure decreases an area of the first surface of the manifold, an area of the second surface of the manifold, or both.
61. The method of any one of clauses 57-60, wherein applying negative pressure opens the fluid restrictions in the fluid management layer.
62. The method of any one of clauses 57-61, further comprising fluidly coupling a fluid container between the dressing and the negative-pressure source, and transferring exudate from the dressing to the fluid container.
63. The method of any one of clauses 57-62, further comprising applying a manifold between the dressing and the surface wound.

## Claims

1. A dressing for treating a tissue site with negative pressure, the dressing comprising:
a tissue interface layer comprising a polymer film having a plurality of fluid restrictions extending through the polymer film and configured to deform; and
a manifold layer coupled to the fluid management layer, the manifold layer having a first surface facing the fluid management layer, a second surface opposite the first surface, and a thickness extending between the first surface and the second surface, the manifold comprising an expanded foam and configured to exhibit a radial collapse such that the an area of the first surface, the second surface, or both, when the manifold layer is subjected to negative pressure, is decreased relative to the area of the first surface or the second surface when the manifold layer is not subjected to negative pressure.

2. The dressing of claim 1, wherein the expanded foam material is formed from a process comprising:
impregnation of a polymeric material with an inert gas at high heat and pressure to form an impregnated polymeric material; and
expansion of the impregnated polymeric material to form the expanded foam material.

3. The dressing of claim 2, wherein the polymeric material comprises a cross-linked ethylene-vinyl acetate copolymer, or a cross-linked polyolefin, or a cross-linked polyethylene, or a cross-linked ethyl-methyl-acrylate copolymer.

4. The dressing of one of claims 2-3, wherein the polymeric material comprises an antimicrobial material and/or an absorbent.

5. The dressing of one of claims 2-4, wherein the inert gas comprises at least 95% by weight of nitrogen gas.

6. The dressing of any preceding claim, wherein the expanded foam comprises a cross-linked ethylene-vinyl acetate copolymer, a cross-linked polyolefin, a cross-linked polyethylene, or a cross-linked ethyl-methyl-acrylate copolymer.

7. The dressing of any preceding claim, wherein the expanded foam comprises an antimicrobial material incorporated within the expanded foam.

8. The dressing of any preceding claim, wherein the expanded foam comprises a closed-cell foam.

9. The dressing of any preceding claim, wherein the manifold layer further comprises a plurality of collapsible apertures extending at least partially through the thickness of the manifold layer from the first surface, the collapsible apertures configured to deform in response to a negative pressure applied to the manifold layer,
in particular wherein each of the plurality of collapsible apertures comprises:
a length parallel to the first surface of the manifold layer; and
a width parallel to the first surface of the manifold layer and perpendicular to the length,
wherein the length of each of the plurality of collapsible aperture is greater than its width,
in particular wherein the length is at least 1.5 times the width, and/or wherein the length and the width of each of the collapsible apertures is substantially constant, and/or wherein each of the plurality of collapsible apertures are rectangular, elongate, diamond-shaped, square, oval, ovoid, irregular, polygonal, or amorphous.

10. The dressing of claim 9, wherein the plurality of collapsible apertures includes a first portion of collapsible apertures having a first orientation and a second portion of collapsible apertures having a second orientation,
in particular wherein the first orientation deviates from the second orientation by between 75 degrees and 105 degrees, in particular wherein the first orientation is substantially perpendicular to the second orientation.

11. The dressing of claim 10, wherein the first portion of collapsible apertures is arranged in a first plurality of rows and the second portion of collapsible apertures is arranged in a second plurality of rows,
in particular wherein each of the first plurality of rows is adjacent to one of the second plurality of rows.

12. The dressing of one of claims 10-11, wherein a perpendicular bisector of the length of one of the first portion of collapsible apertures extends through at least one of the second portion of collapsible apertures.

13. The dressing of one of claims 10-12, wherein a perpendicular bisector of the length of one of the first portion of collapsible apertures extends through a mid-point of the width of one of the second portion of collapsible apertures.

14. The dressing of any preceding claim, wherein the manifold layer further comprises a plurality of fluid apertures extending through the surface of the manifold between the first surface and the second surface, wherein the fluid apertures are configured to exhibit little or no deformation in response to a negative pressure relative to the plurality of collapsible apertures.

15. A system for treating a tissue site, the system comprising:
the dressing of any preceding claim; and
a negative-pressure source fluidly coupled to the dressing,
optionally further comprising a fluid container fluidly coupled between the dressing and the negative-pressure source.
